(19) Europäisches Patentamt
European Patent Office
Office européen des brevets

(11) Veröffentlichungsnummer: **0 038 302**
**A2**

# (12) EUROPÄISCHE PATENTANMELDUNG

(21) Anmeldenummer: **81810137.0**

(22) Anmeldetag: **08.04.81**

(51) Int. Cl.³: **C 07 D 307/93**, A 01 N 43/12

(30) Priorität: **14.04.80 CH 2858/80**

(43) Veröffentlichungstag der Anmeldung: **21.10.81**
**Patentblatt 81/42**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Baumann, Marcus, Dr., Rührbergerstrasse 6, CH-4058 Basel (CH)**
Erfinder: **Bühler, Niklaus, Dr., Gartenweg 30, CH-4310 Rheinfelden (CH)**
Erfinder: **Bellus, Daniel, Dr., Unterm Schellenberg 81, CH-4125 Riehen (CH)**

## (54) Cyclobutandicarbonsäure-Isoimide, Verfahren zu deren Herstellung und deren Verwendung als Fungizid.

(57) Cyclobutandicarbonsäure-isoimide der Formel I

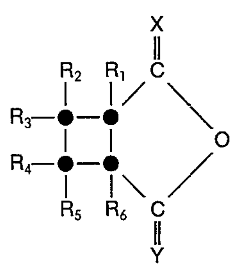

(I),

worin eines von X und Y = Sauerstoff und das andere

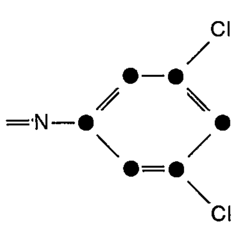

bedeutet und $R_1$ bis $R_6$ unabhängig voneinander Methyl oder Äthyl bedeuten, $R_2$ und $R_3$ unabhängig voneinander Wasserstoff, Fluor oder Methyl darstellen oder aber $R_2$ $-OCOCH_3$ oder Chlor und gleichzeitig $R_3$ Wasserstoff bedeuten, und $R_4$ Wasserstoff, Methyl oder Chlor ist und $R_5$ Wasserstoff oder Methyl darstellt, wobei auch $R_3$ und $R_4$ zusammen eine zusätzliche Bindung im Vierring bilden können, eignen sich zur Bekämpfung verschiedenartiger phytopathogener Pilze. Sie können durch Cyclisierung der entsprechenden Amidcarbonsäuren bei Temperaturen zwischen etwa —20° und +100° C in Gegenwart von Dehydratisierungsmitteln, wie Acetanhydrid oder N,N'-Dicyclohexylcarbodiimid hergestellt werden.

EP 0 038 302 A2

CIBA-GEIGY AG
Basel (Schweiz)

5-12805/ZFO

Cyclobutandicarbonsäure-isoimide, Verfahren zu deren Herstellung und deren Verwendung als Fungizid

Die vorliegende Erfindung betrifft neue Cyclobutandicarbonsäure-isoimide, ein Verfahren zu deren Herstellung, fungizide Mittel, die solche Verbindungen als Aktivsubstanz enthalten, sowie die Verwendung dieser Verbindungen zur Bekämpfung von Pilzen.

Aus der japanischen Offenlegungsschrift 74/71 141 und der europäischen Patentveröffentlichung 0001395 ist bekannt, dass am Cyclobutanring gegebenenfalls substituierte 3,5-Dihalogenphenylcyclobutandicarbonsäureimide antimikrobielle, u.a. auch fungizide Eigenschaften aufweisen und sich beispielsweise zur Bekämpfung von Pilzerkrankungen am Reis eignen.

Es wurden nun neue Cyclobutandicarbonsäure-isoimide der Formel I

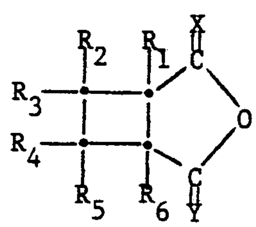

(I),

gefunden, worin eines von X und Y = Sauerstoff und das andere

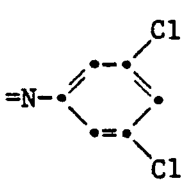

darstellt,

$R_1$ und $R_6$ unabhängig voneinander Methyl oder Aethyl bedeuten, $R_2$ und $R_3$ unabhängig voneinander Wasserstoff, Fluor oder Methyl darstellen oder aber $R_2$ $-OCOCH_3$ oder Chlor und gleichzeitig $R_3$ Wasserstoff bedeuten, und $R_4$ Wasserstoff, Methyl oder Chlor ist und $R_5$ Wasserstoff

oder Methyl darstellt, wobei auch $R_3$ und $R_4$ zusammen eine zusätzliche Bindung im Vierring bilden können.

Bevorzugt sind Verbindungen der Formel I, worin $R_1$ und $R_6$ je Methyl, $R_2$ und $R_3$ unabhängig voneinander Wasserstoff, Fluor oder Methyl und $R_4$ und $R_5$ unabhängig voneinander Wasserstoff oder Methyl bedeuten. Besonders bevorzugt sind Verbindungen der Formel I, worin $R_1$ und $R_6$ je Methyl, $R_2$ und $R_3$ unabhängig voneinander Wasserstoff oder Fluor und $R_4$ und $R_5$ je Wasserstoff darstellen. Ganz besonders bevorzugt ist die Verbindung der Formel I, worin $R_1$ und $R_6$ je Methyl und $R_2$, $R_3$, $R_4$ und $R_5$ je Wasserstoff bedeuten.

Die Verbindungen der Formel I weisen ausgezeichnete fungizide Eigenschaften auf und besitzen insbesondere eine ausgeprägte Botrytis-Wirkung. Botrytis spp. (B. cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar.

Die Verbindungen der Formel I besitzen ein für die praktischen Bedürfnisse sehr günstiges Wirkungsspektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, vor allem Steinobst, Zierpflanzen, Reben, Hopfen, Gurkengewächse (Gurken, Kürbis, Melonen), Solanaceen, wie Kartoffeln, Tomaten und Tabak, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae, Fusarium, Helminthosporium); Basidiomycetes, wie vor allem Rostpilze (z.B. Puccinia, Tilletia); Fungi

imperfecti (z.B. Moniliales u.a., Cercospora, Sclerotinia sowie Botrytis und Piricularia) und die der Klasse der Phycomycetes angehörenden Oomycetes, wie Phytophthora oder Plasmopara. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Verbindungen der Formel I können z.B. dadurch hergestellt werden, dass man eine Verbindung der Formel II

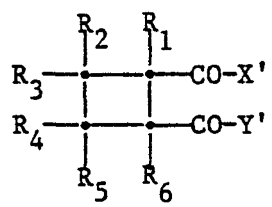 (II),

worin eines von X' und Y' 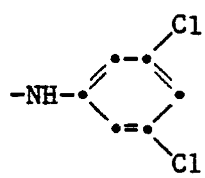 und das andere -OH bedeuten und $R_1$ bis $R_6$ die unter Formel I angegebene Bedeutung haben, bei einer Temperatur zwischen -20°C und +100°C, bevorzugt zwischen 0°C und 40°C in Gegenwart eines Dehydratisierungsmittels und vorzugsweise in Gegenwart eines inerten organischen Lösungsmittels zum Isoimid der Formel I cyclisiert.

Isoimide sind im allgemeinen thermodynamisch labil und lagern sich relativ leicht in die entsprechenden Imide um (vgl. z.B. deutsche Offenlegungsschrift 2.715.435). Ueberraschenderweise lassen sich aber diese Amidcarbonsäuren der Formel II unter den angegebenen Reaktionsbedingungen in stabile Isoimide der Formel I überführen.

Als Dehydratisierungsmittel kommen z.B. in Betracht:
Anhydride von gegebenenfalls durch Halogenatome oder $C_{1-4}$-Alkylgruppen substituierten aliphatischen $C_{2-5}$-Monocarbonsäuren, wie Essigsäure-,

Propionsäure-, Buttersäure- und Valeriansäureanhydrid, Trichlor-, Trifluor-, Trimethyl-, Triäthyl- und Tri-n-butylessigsäureanhydrid; Carbodiimide, wie N,N'-Diisopropylcarbodiimid und N,N'-Dicyclohexylcarbodiimid; sowie Keten. Ferner können Gemische von tertiären Aminen und den oben erwähnten Anhydriden oder Gemische von tertiären Aminen und gegebenenfalls halogenierten Acetylhalogeniden, wie Acetylchlorid, Chloracetylchlorid, Dichloracetylchlorid und Trifluoracetylchlorid, eingesetzt werden. Geeignete tertiäre Amine sind z.B. N,N-Dimethylanilin, N-Methyl-N-äthylanilin, N-Methyl- oder N-Aethylmorpholin, Pyridin, Chinuclidin oder N,N'-Dimethylpiperazin und insbesondere Trialkylamine mit je 1-8 C-Atomen in den Alkylgruppen, wie Triäthylamin, Tri-n-butylamin, Tri-(2-äthyl-n-hexyl)-amin und Tri-n-octylamin.

Bevorzugte Dehydratisierungsmittel sind Acetanhydrid, N,N'-Dicyclohexylcarbodiimid und Keten. Dabei kann überschüssiges Acetanhydrid auch als Lösungsmittel dienen.

Geeignete inerte organische Lösungsmittel zur Durchführung der Cyclisierung sind z.B. gegebenenfalls chlorierte aromatische Kohlenwasserstoffe, wie Benzol, Toluol, Xylole und Chlorbenzol; chlorierte aliphatische Kohlenwasserstoffe, wie Chloroform, Methylenchlorid und 1,2-Dichloräthan; aliphatische und cycloaliphatische Ketone, wie Aceton, Methyläthylketon und Cyclohexanon; sowie Dialkyläther mit je 2-6 C-Atomen in den Alkylteilen und cyclische Aether wie Tetrahydrofuran und Dioxan. Besonders bevorzugt wird die Umsetzung in Acetanhydrid oder Toluol durchgeführt.

Nach Beendigung der Umsetzung können die Isoimide der Formel I auf übliche Weise isoliert werden, z.B. indem man das Lösungsmittel abdestilliert und den Rückstand aus einem geeigneten Lösungsmittel, wie Methanol, Essigsäureäthylester oder Aceton, umkristallisiert.

Die Amidsäuren der Formel II können z.B. dadurch erhalten werden, dass man eine Verbindung der Formel III

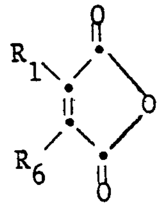

(III)

vorzugsweise in Gegenwart eines Sensibilisators durch Lichteinwirkung mit einer Verbindung der Formel IV

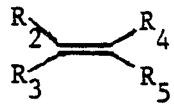

(IV)

zu einer Verbindung der Formel V

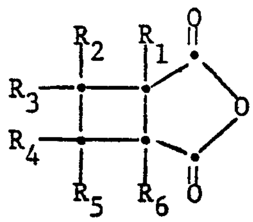

(V)

umsetzt und diese mit 3,5-Dichloranilin reagieren lässt. Dabei haben $R_1$ bis $R_6$ die unter Formel I angegebene Bedeutung.

Die obige photochemische [2+2]-Cycloaddition der Verbindungen der Formel IV an Verbindungen der Formel III wird mit Vorteil in Gegenwart eines inerten organischen Lösungsmittels bei Temperaturen zwischen etwa -80°C und +30°C, bevorzugt -40°C bis 0°C durchführt.

Geeignete inerte organische Lösungsmittel sind die für die Cyclisierungsreaktion genannten. Bevorzugt als Lösungsmittel sind chlorierte aliphatische Kohlenwasserstoffe, besonders Dichlormethan.

Als Sensibilisatoren können an sich bekannte Verbindungen eingesetzt werden, bevorzugt solche, die eine $E_T$ (Triplett-Energie) von $\geq$ 230 kJ/mol aufweisen [vgl. Paul S. Engel und Bruce M. Monroe "Advance in Photochemistry", Bd. 8, 297-306, New York 1971]. Beispiele derartiger Sensibilisatoren sind Benzophenon, Acetophenon, Biacetyl.

Im allgemeinen wird der Sensibilisator in einer Menge von etwa 0,5-10 Gew.-%, bezogen auf die Ausgangsverbindung der Formel III, eingesetzt.

Als Lichtquelle für die obige photochemische Umsetzung kann an sich beliebiges Licht mit Wellenlängen unter 5000 Å eingesetzt werden. Geeignete Lichtquellen sind z.B. gegebenenfalls metallatomdotierte Quecksilberhochdrucklampen, Xenondampflampen, Quecksilber-Xenonlampen, Quecksilberniederdruck- oder-mitteldrucklampen, Halogenlampen oder $D_2$-Lampen.

Die Umsetzung der Anhydride der Formel V mit dem 3,5-Dichloranilin wird auf an sich bekannte Weise vorgenommen, zweckmässig in organischem Medium bei Temperaturen zwischen etwa 10 und 40°C.

Die Verbindungen der Formel I können für sich allein oder bevorzugt zusammen mit geeigneten Trägermaterialien und/oder anderen Zuschlagstoffen, besonders oberflächenaktiven Mitteln, verwendet werden. Geeignete Trägermaterialien und Zuschlagstoffe können fest oder flüssig sein und entsprechen den in der Formulierungstechnik üblichen Stoffen, wie z.B. natürlichen oder regenerierten mineralischen Stoffen, Lösungs-, Dispergier-, Netz-, Haft-, Verdickungs-, Binde- oder Düngemitteln. Wirkstoffe der Formel I können im Gemisch mit anderen pestiziden oder mit pflanzenwuchsverbessernden Präparaten verwendet werden.

Der Gehalt an Wirkstoff in handelsfähigen Mitteln liegt zwischen 0,1 bis 90 Gew.-%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10 Gew.-%); Granulate, Umhüllungsgranulate, Imprägnierungsgranulate und Homogengranulate, Pellets (Körner) (1 bis 80 Gew.-%);

Flüssige Aufarbeitungsformen:

a) in Wasser dispergierbare Wirkstoffkonzentrate:

Spritzpulver (wettable powders) und Pasten (25-90 Gew.-% in der Handelspackung, 0,01 bis 15 Gew.-% in gebrauchsfertiger Lösung); Emulsions- und Lösungskonzentrate (10 bis 50 Gew.-% in der Handelpackung; 0,01 bis 15 Gew.-% in gebrauchsfertiger Lösung);

b) Lösungen (0,1 bis 20 Gew.-%); Aerosole.

## Herstellungsbeispiele

### Beispiel 1

a) In einer Tieftemperaturbelichtungsanlage, ausgerüstet mit einem 125 W Quecksilberbrenner in gekühltem Tauchbad, Rührer und Gaseinleitungsrohr, werden 15 g (0,12 Mol) Dimethylmaleinsäureanhydrid und 1 g Benzophenon (0,0054 Mol) in 300 ml Dichlormethan gelöst und durch ein externes Kühlbad mit einer Isopropanol-Trockeneis-Mischung auf -60 bis -70°C abgekühlt, eine Stunde mit Aethylen begast und anschliessend bei dieser Temperatur 12 Stunden unter schwachem Aethylenstrom bestrahlt. Sodann wird das Lösungsmittel abgedampft, und das Rohprodukt wird aus Dichlormethan/n-Hexan umkristallisiert. Man erhält 10 g 1,2-Dimethyl-cyclobutan-1,2-dicarbonsäureanhydrid (54% d.Th.) in Form weisser Kristalle; Smp. 84-86°C.

b) 15,4 g (0,1 Mol) 1,2-Dimethyl-cyclobutan-1,2-dicarbonsäureanhydrid werden in 100 ml Toluol gelöst und mit 16,2 g (0,1 Mol) 3,5-Dichloranilin versetzt. Nach 16-stündigem Rühren bei 25°C wird die ausgefallene farblose Amidcarbonsäure abgetrennt. Man erhält 27,8 g 1-(3,5-Dichlorphenylcarbamoyl)-1,2-dimethylcyclobutan-1-carbonsäure (88% d.Th.) vom Smp. 164°C.

c) 31,6 g 1-(3,5-Dichlorphenylcarbamoyl)-1,2-dimethylcyclobutan-1-carbonsäure werden mit 100 ml Acetanhydrid bei 20°C während 4 Stunden verrührt. Nach kurzer Zeit entsteht eine klare Lösung. Das Acetanhydrid wird im Vakuum bei 40°C abdestilliert, und der Rückstand wird aus Methanol umkristallisiert. Man erhält 22 g 1,2-Dimethyl-cyclobutan-1,2-dicarbonsäure-(3,5-dichlorphenyl)-isoimid, vom Smp. 110-111°C. Ausbeute = 74% d.Th. [Wirkstoff Nr. 1].

NMR-Spektrum (60 MHz, $CHCl_3$, $\delta$ in ppm): 1,35 (s, 3H, $-CH_3$); 1,45 (s, 3H, $-CH_3$); 2,0-2,7 (m, 4H, $-CH_2CH_2-$); 6,9-7,1 (m, 3H, aromat.).

IR-Spektrum (KBr): 1840 $cm^{-1}$ (s) und 1730 $cm^{-1}$ (vs).

Elementaranalyse:

| | | | |
|---|---|---|---|
| berechnet | C 56,4 % | H 4,4 % | Cl 23,8 % |
| gefunden | C 56,5 % | H 4,4 % | Cl 24,0 %. |

Beispiel 2

15,8 g 1-(3,5-Dichlorphenylcarbamoyl)-1,2-dimethylcyclobutan-1-carbonsäure werden in 100 ml Toluol suspendiert. Dazu gibt man 11 g N,N'-Dicyclohexylcarbodiimid, gelöst in 100 ml Toluol. Die Temperatur des Reaktionsgemisches steigt dabei auf 30°C. Nach 3 Stunden Rühren bei 20-25°C wird vom gebildeten N,N'-Dicyclohexylharnstoff abfiltriert, und das Filtrat wird im Vakuum bei 40°C zur Trockne eingedampft. Man erhält 14,8 g (100% d.Th.) 1,2-Dimethyl-cyclobutan-1,2-dicarbonsäure-(3,5-dichlorphenyl)-isoimid vom Smp. 110-111°C, dessen spektroskopische Daten mit denjenigen der gemäss Beispiel 1 erhaltenen Verbindung übereinstimmen.

Auf eine zu Beispiel 1 oder 2 analoge Art werden auch folgende Verbindungen hergestellt:

Nr. 2 1,2,3- bzw. 1,2,4-Trimethyl-cyclobutan-1,2-dicarbonsäure-(3,5-dichlorphenyl)-isoimid, Smp. 100-102°C;

Nr. 3 1,3-Dimethyl-3,3-(4,4-)difluor-cyclobutan-1,2-dicarbonsäure-(3,5-dichlorphenyl)-isoimid, Smp. 68-74°C;

Nr. 4 1,2-Dimethyl-cyclobuten(3,4)-1,2-dicarbonsäure-(3,5-dichlorphenyl)-isoimid, Smp. 108-110°C.

Die Verbindungen Nr. 2 und 3 stellen je Gemische von Stereo- und Regio-Isomeren dar.

Die Wirkstoffe der Formel I vorliegender Erfindung können beispielsweise wie folgt formuliert werden:

Beispiel 3:

Stäubemittel: Zur Herstellung eines 5%igen Stäubemittels werden 5 Teile eines Wirkstoffes Nr. 1 bis Nr. 4 mit 95 Teilen Talkum anwendungsfertig vermahlen.

Beispiel 4:

Granulat: Zur Herstellung eines 5%igen Granulates werden die folgenden Stoffe verwendet:

5 Teile Wirkstoff Nr. 1,
0,25 Teile epoxidiertes Pflanzenöl
0,25 Teile Cetylpolyglykoläther,
3,50 Teile Polyäthylenglykol
91 Teile Kaolin (Korngrösse 0,3-0,8 mm).

Die Aktivsubstanz wird mit dem epoxidierten Pflanzenöl vermischt und mit 6 Teilen Aceton gelöst; hierauf werden Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 5:

Spritzpulver: Zur Herstellung eines a) 40%igen b) 25%igen c) 10%igen Spritzpulvers werden folgende Bestandteile verwendet:

| | | |
|---|---|---|
| a) | 40 | Teile eines Wirkstoffs Nr. 1 bis Nr. 4 |
| | 5 | Teile Ligninsulfonsäure-Natriumsalz, |
| | 1 | Teil Dibutylnaphthalinsulfonsäure-Natriumsalz, |
| | 54 | Teile Kieselsäure; |
| b) | 25 | Teile eines Wirkstoffs Nr. 1 bis Nr. 4 |
| | 4,5 | Teile Calcium-Ligninsulfonat, |
| | 1,9 | Teile Champagne-Kreide/Hydroxyäthylcellulose-Gemisch (1:1), |
| | 1,5 | Teile Natriumdibutylnaphthalinsulfonat, |
| | 19,5 | Teile Kieselsäure, |
| | 19,5 | Teile Champagne-Kreide, |
| | 28,1 | Teile Kaolin; |
| c) | 10 | Teile eines Wirkstoffs Nr. 1 bis Nr. 4, |
| | 3 | Teile Gemisch der Natriumsalze von gesättigten Fettalkoholsulfaten, |
| | 5 | Teile Naphthalinsulfonsäure/Formaldehyd-Kondensat, |
| | 82 | Teile Kaolin. |

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 6:

Emulgierbare Konzentrate: Zur Herstellung eines 25%igen emulgierbaren Konzentrates werden folgende Stoffe verwendet:

25 Teile eines Wirkstoffs Nr. 1 bis Nr. 4
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

Biologische Beispiele

Beispiel 7:

a) Wirkung gegen Botrytis cinerea auf Bohnen (Vicia faba)
Residual-protektive Wirkung: ca. 10 cm hohe Bohnen-Pflanzen wurden mit einer aus Spritzpulver des Wirkstoffes hergestellten Spritzbrühe besprüht, enthaltend 0,02% bzw. 0,006% Aktivsubstanz. Nach 48 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21°C erfolgte die Beurteilung des Pilzbefalls. Die Wirkstoffe Nr. 1 bis 4 erzielten in 0,02%iger Konzentration eine vollständige Verhütung des Krankheitsbefalls. Die Wirkstoffe Nr. 1, 2 und 4 verhüteten den Befall auch noch in 0,006%iger Konzentration.

b) Wirkung gegen Sclerotinia fructigena auf Aepfeln: Künstlich verletzte Aepfel wurden mit Sclerotinia fructigena infiziert, indem auf jede Verletzungsstelle ein Tropfen Myzelsuspension pipettiert wurde. Nach Abtrocknen des Inokulum-Tropfens wurden die Aepfel mittels einer

Spritzpulversuspension der Wirksubstanz (0,02% Aktivsubstanz) besprüht. Die behandelten Früchte wurden in Plastikbehälter gelegt und während 14 Tagen bei 20-22°C gelagert. Zur Auswertung wurde die Anzahl der angefaulten Verletzungsstellen bestimmt.

Die erfindungsgemässen Wirkstoffe Nr. 1 bis Nr. 4 verhüteten den Krankheitsbefall vollständig.

c) Wirkung gegen Monilinia auf Kirschblüten oder Pfirsichblüten (Monilinia-Befall an Steinobst): Einzelne voll erblühte Steinobstzweige werden mit einer aus einem Emuslionskonzentrat hergestellten Spritzbrühe (enthaltend 0,025% Aktivsubstanz) besprüht. Einige Stunden später werden die Blütenstände abgeschnitten, mit dem Stiel in den nassen Sand von Plastikschalen gesteckt und mit einer Sporensuspension inokuliert. Die Schalen werden dann lose mit transparenter Plastikfolie bedeckt und 2 Tage bei Raumtemperatur gehalten. Mit der Zahl von befallenen Blüten wird das Ausmass des Krankheitsbefalls bestimmt, wobei je 40 Blüten pro Wirkstoff verwendet werden. Die Verbindungen Nr. 2 und 3 bewirkten eine Reduktion des Krankheitsbefalls auf unter 20%. Die Verbindungen Nr. 1 und 4 verhüteten den Krankheitsbefall vollständig.

Patentansprüche

1. Verbindungen der Formel I

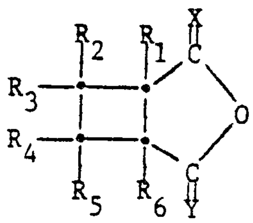

(I),

worin eines von X und Y = Sauerstoff und das andere

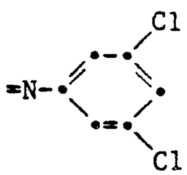

darstellt,

$R_1$ und $R_6$ unabhängig voneinander Methyl oder Aethyl bedeuten, $R_2$ und $R_3$ unabhängig voneinander Wasserstoff, Fluor oder Methyl darstellen oder aber $R_2$ $-OCOCH_3$ oder Chlor und gleichzeitig $R_3$ Wasserstoff bedeuten, und $R_4$ Wasserstoff, Methyl oder Chlor ist und $R_5$ Wasserstoff oder Methyl darstellt, wobei auch $R_3$ und $R_4$ zusammen eine zusätzliche Bindung im Vierring bilden können.

2. Verbindungen der Formel I nach Anspruch 1, worin $R_1$ und $R_6$ je Methyl, $R_2$ und $R_3$ unabhängig voneinander Wasserstoff, Fluor oder Methyl und $R_4$ und $R_5$ unabhängig voneinander Wasserstoff oder Methyl bedeuten.

3. Verbindungen der Formel I nach Anspruch 1, worin $R_1$ und $R_6$ je Methyl, $R_2$ und $R_3$ unabhängig voneinander Wasserstoff oder Fluor und $R_4$ und $R_5$ je Wasserstoff bedeuten.

4. Verbindung der Formel I nach Anspruch 1, worin $R_1$ und $R_6$ je Methyl und $R_2$, $R_3$, $R_4$ und $R_5$ je Wasserstoff bedeuten.

5. Verfahren zur Herstellung einer Verbindung der Formel I nach Anspruch 1, dadurch gekennzeichnet, dass man eine Verbindung der

Formel II

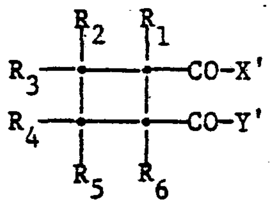 (II),

worin eines von X' und Y' 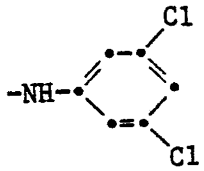 und das andere -OH bedeutet und $R_1$ bis $R_6$ die im Anspruch 1 angegebene Bedeutung haben, bei einer Temperatur zwischen -20°C und +100°C in Gegenwart eines Dehydratisierungsmittels zu einem Isoimid der Formel I cyclisiert.

6. Verfahren nach Anspruch 5, dadurch gekennzeichnet, dass man die Cyclisierung bei einer Temperatur zwischen 0°C und 40°C vornimmt.

7. Verfahren nach Anspruch 5, dadurch gekennzeichnet, dass man die Cyclisierung in Gegenwart eines inerten organischen Lösungsmittels vornimmt.

8. Verfahren nach Anspruch 5, dadurch gekennzeichnet, dass man als Dehydratisierungsmittel N,N'-Dicyclohexylcarbodiimid, Acetanhydrid oder Keten verwendet.

9. Verfahren nach Anspruch 7, dadurch gekennzeichnet, dass man als inertes organisches Lösungsmittel Acetanhydrid oder Toluol verwendet.

10. Fungizides Mittel, enthaltend als Aktivsubstanz eine Verbindung der Formel I nach einem der Ansprüche 1 bis 4 zusammen mit einem geeigneten Trägermaterial und/oder einem oberflächenaktiven Mittel.

11. Verwendung einer Verbindung der Formel I nach einem der Ansprüche 1 bis 4 zur Bekämpfung von Pilzen und zur Verhütung von Pilzbefall.